(19) Europäisches Patentamt
European Patent Office
Office européen des brevets

(11) Veröffentlichungsnummer: **0 023 685**
**A2**

(12) # EUROPÄISCHE PATENTANMELDUNG

(21) Anmeldenummer: **80104404.1**

(22) Anmeldetag: **26.07.80**

(51) Int. Cl.³: **A 61 K 6/08**

(30) Priorität: **07.08.79 DE 2931926**

(43) Veröffentlichungstag der Anmeldung: **11.02.81**
**Patentblatt 81/6**

(84) Benannte Vertragsstaaten: **BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Schmitz-Josten, Robert, Dr., Gerstenkamp 6, D-5000 Koeln 80 (DE)**
Erfinder: **Borgardt, Manfred, Dr., Rödiger-Strasse 17, D-5600 Wuppertal (DE)**
Erfinder: **Schulz, Hans-Hermann, Am Neuland Kreuz 23, D-5672 Leichlingen (DE)**
Erfinder: **Walkowiak, Michael, Dr., Albert-Magnus-Strasse 10, D-5090 Leverkusen 1 (DE)**

(54) **Dentalmassen.**

(57) Massen, welche polymerisierbare (Meth)-acrylsäureester der Formel

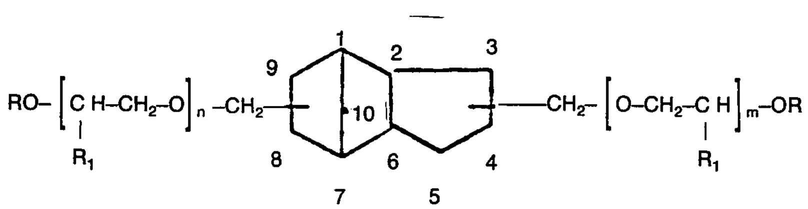

(I)

in welcher R, $R_1$, n und m die in der Beschreibung angegebene Bedeutung besitzen, enthalten, eignen sich gut für die Verwendung in der Zahnheilkunde.

EP 0 023 685 A2

0023685

BAYER AKTIENGESELLSCHAFT 5090 Leverkusen, Bayerwerk
Zentralbereich
Patente, Marken und Lizenzen Si/AB -c 6. 08. 79

Dentalmassen

Gegenstand der Erfindung sind Dentalmassen, insbesondere für Zahnfüllungen und Zahnersatzteile.

Es ist bekannt, polymerisierbare Monomere, insbesondere Ester der Acryl- oder Methacrylsäure für die Herstellung von Zahnfüllungen, Zahnreparaturmaterialien, Zahnversiegelungsmassen, Zahnprothesen und anderen Dentalmassen zu verwenden.

Zur Erhöhung der mechanischen Eigenschaften, wie Druckfestigkeit oder Abriebsfestigkeit werden häufig polyfunktionelle Derivate der Acryl- oder Methacrylsäure verwendet. Für die Herstellung von Zahnfüllmassen hat sich beispielsweise das in der US-PS 3 066 112 beschriebene Umsetzungsprodukt aus 2 Mol Glycidylmethacrylat und 1 Mol Bisphenol A (kurz als BIS-GMA bezeichnet) bewährt, welches zusammen mit Quarzglas oder ähnlichen verarbeitet wird. Solche auch als Composit-Zahnfüllmassen bezeichneten Produkte enthalten meist Monomeren-Gemische und bis zu 80 % eines mit Haftvermittlern vorbehandelten anorganischen Füllstoffs wie Quarz oder Silikate. Häufig werden zur Erhöhung der Röntgenopazität noch Silikate des Bariums, Lanthans, Zirkons oder anderer Metalle mit hoher Ordnungszahl zugesetzt.

Die Aushärtung der Massen erfolgt durch radikalbildende Reaktionen, wie z.B. durch Redoxpolymerisation, oder durch Photopolymerisation in Gegenwart geeigneter Photoinitiatoren, wie z.B. die US-PS 3 629 187 und die US-PS 3 709 866 lehren.

Solche Komposit-Zahnfüllmassen lassen sich im Gegensatz zu Amalgamfüllungen in jedem gewünschten Farbton herstellen, was besonders für den Frontzahnbereich von erheblicher Bedeutung ist.

Es sind noch eine Vielzahl von anderen Monomeren als BIS-GMA auf ihre Eignung als polymerisierbare Bindemittel für Dentalmassen, insbesondere für Zahnfüll- und Reparaturmassen vorgeschlagen worden, doch sind sie in vielen Eigenschaften noch verbesserungswürdig; die Amalgamfüllungen sind den Komposits insbesondere in der Biege- und Abriebsfestigkeit noch überlegen.

Es wurde nun überraschenderweise gefunden, daß die Acryl- oder Methacrylsäureester des oxyalkylierten Bis-hydroxy-methyltricyclo-$[5.2.1.0.^{2.6}]$decans Id besonders geeignete Bindemittel für Dentalmassen darstellen. Sie lassen sich nach an sich bekannten Methoden durch Umsetzung des Tricyclodecan-derivats Ia mit Alkylenoxiden zu Ic und anschließende Veresterung mit Acryl- oder Methacrylsäure herstellen.

Das Bis-hydroxymethyl-tricyclo$[5.2.1.0.^{2.6}]$decan Ia ist im Handel unter dem Namen TCD-DM erhältlich. Wie Formel

Ia zeigt, ist es ein Isomerengemisch, wobei sich die Hydroxymethylgruppen sowohl in 3- oder 4-Stellung als auch in 8- oder 9-Stellung befinden können. Durch Anlagerung von Alkylenoxiden, speziell von Ethylenoxid, Propylenoxid und Butylenoxid in Gegenwart basischer Katalysatoren erhält man die Etheralkohole der allgemeinen Formel Ic, welche dann in die Acrylsäure- oder Methacrylsäureester Id umgewandelt werden.

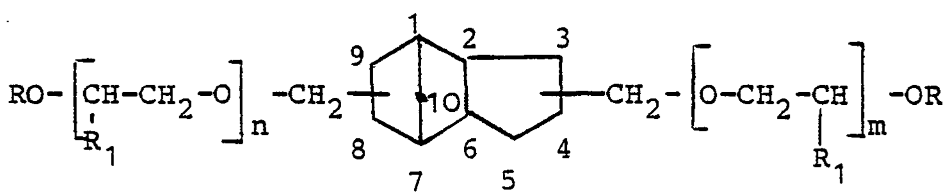

I a: $R = H;\ n + m = O$

I b: $R = CH_2 = CH - CO$ oder $CH_2 = \underset{CH_3}{C} - CO;\ n + m = O$

I c: $R = H;\ n + m = 1\text{-}10;\ R_1 = H,\ CH_3$ oder $C_2H_5$

I d: $R = CH_2 = CH - CO$ oder $CH_2 = \underset{CH_3}{C} - CO;$
$R_1 = H,\ CH_3$ oder $C_2H_5;$
$n + m = 1 - 10.$

Die ethergruppenfreien Acryl- und Methacrylsäureester Ib sowie ihre Verwendung für Dentalmassen sind aus der DT-OS 2 200 021 und der DT-OS 2 816 823 bekannt, während die Ester der Formel Id noch nicht beschrieben sind. (Sie und ihre Herstellung sind jedoch Gegenstand einer eigenen Patentanmeldung vom gleichen Tage).

Sie bieten durch ihre Vielfacht bessere Auswahlmöglichkeiten an dem jeweiligen Verwendungszweck besser anzupassenden Monomeren als die Acrylate Ib. Insbesondere ist ihr Polymerisationsschrumpf geringer. Ferner erfolgt durch den höheren Sauerstoffgehalt eine zwar geringe, aber gezielt erhöhte Wasseraufnahme, welche den Polymerisationsschrumpf weiter reduziert. Es wurde ferner festgestellt, daß die aus Id hergestellten Dentalmassen eine höhere Biegefestigkeit besitzen als entsprechende aus Ib hergestellte Massen.

Infolge ihrer niedrigen Viskosität können die Monomeren Id meist ohne weitere Verdünnungsmittel eingesetzt werden, was beim BIS-GMA nicht möglich ist. Sie sind im Gegenteil als Verdünner für hochviskose Dentalbindemittel, wie z.B. BIS-GMA geeignet. Die niedrige Viskosität von Id und ihre guten Benetzungseigenschaften sind besonders vorteilhaft für die Herstellung von Überzugs- und Versiegelungsmassen.

Eine Vermischung mit anderen bekannten Methacrylsäureestern, z.B. zur Einstellung einer bestimmten Viskosität, einer bestimmten Brechungszahl oder eines gewünschten Doppelbindungsgehaltes für den gewünschten Verwendungszweck ist ohne weiteres möglich. Zur Herstellung gemischter Bindemittel mit Id eignen sich besonders polyfunktionelle Monomere, wie Ethylenglykoldimethacrylat, Triethylenglycoldimethacrylat, Trimethylolpropan-trimethacrylat, Pentaerythrit-tetraacrylat,

Butandiol-1,3-dimethacrylat, Hexandiol-1,6-dimethyacrylat. Weitere für Abmischungen mit Id geeignete Monomere sind in der nachstehenden Zusammenstellung formelmäßig aufgeführt:

$R = CH_2 = C(CH_3) - CO -$ oder $CH_2 = CH - CO -$ ,

$R_1 =$ H oder $-CH_2 - OR$ ,

$n = 1 - 4$ und

$m = 0 - 4$ ,

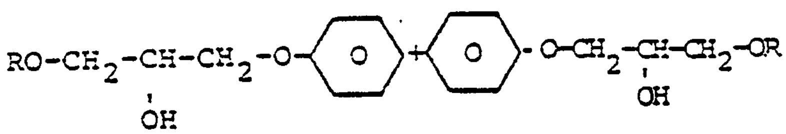

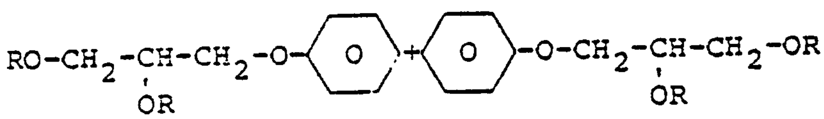

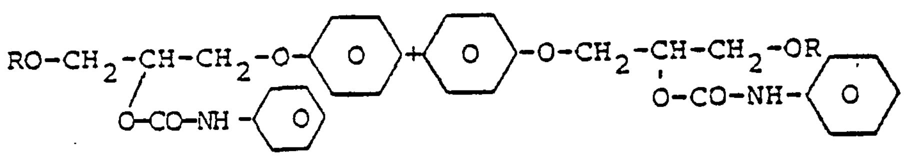

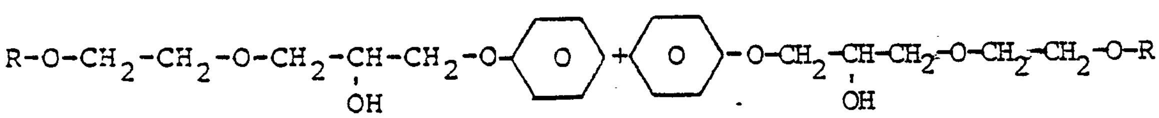

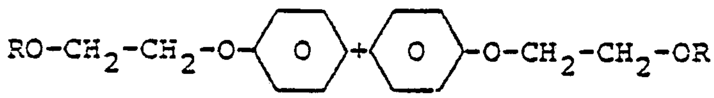

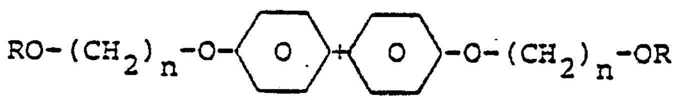

n = > 2

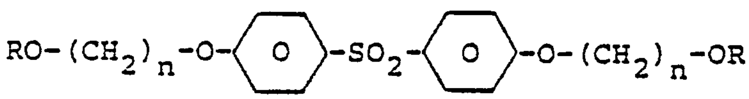

n = 2-4

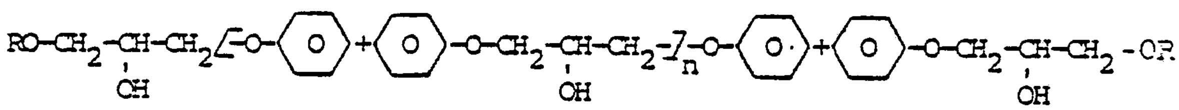

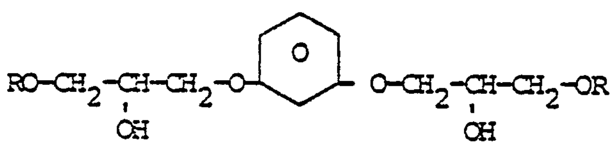

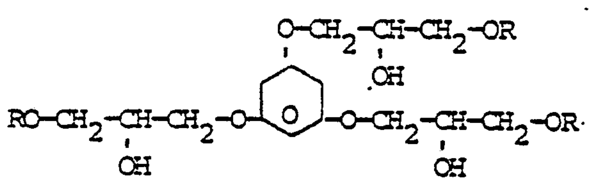

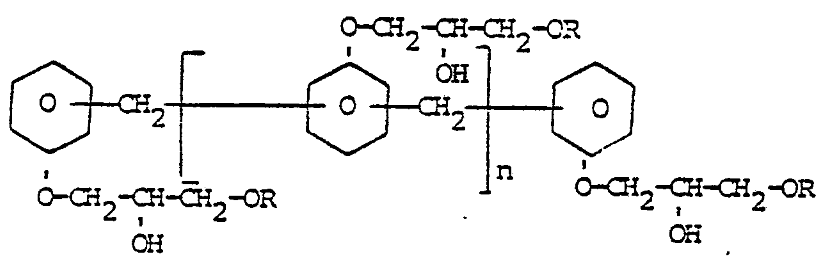

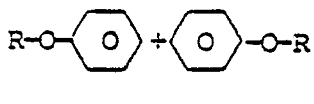

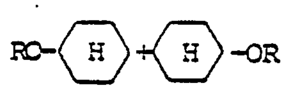

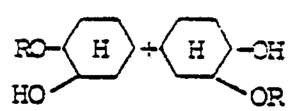

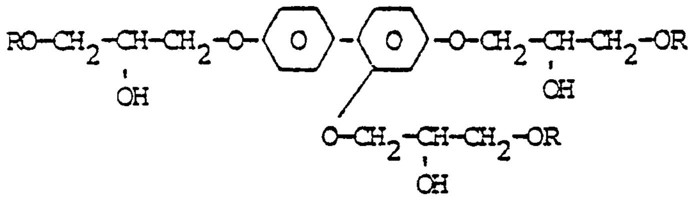

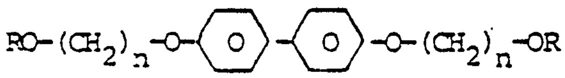

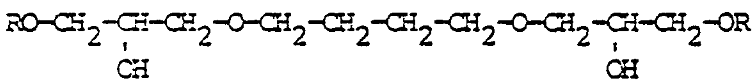

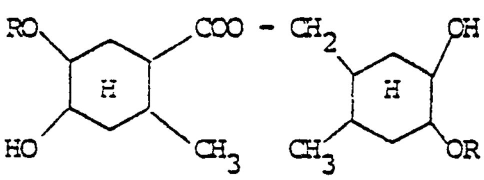

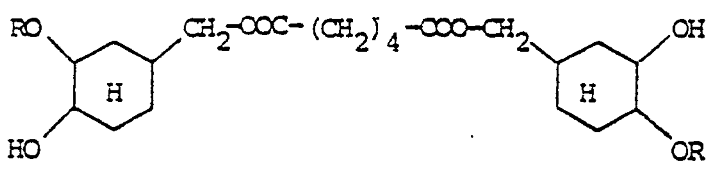

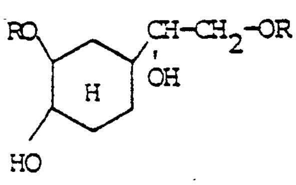

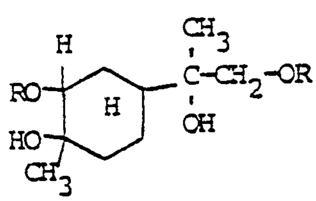

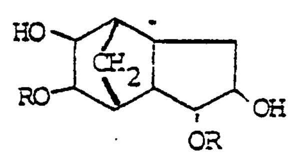

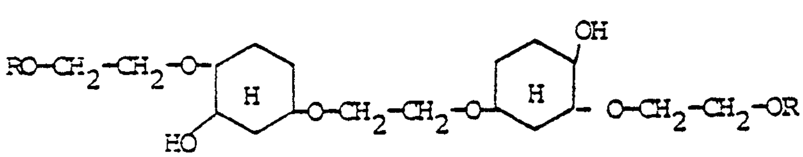

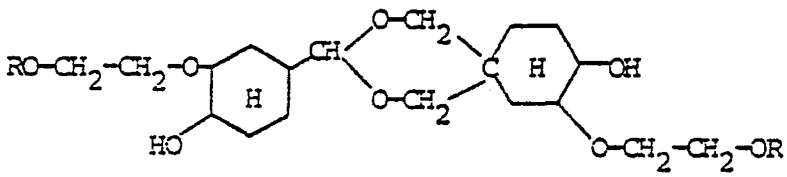

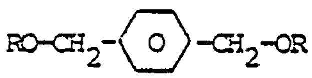

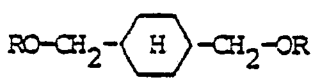

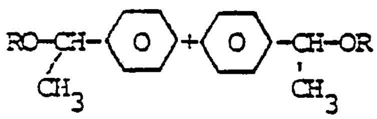

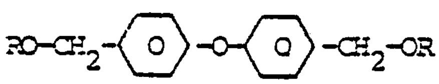

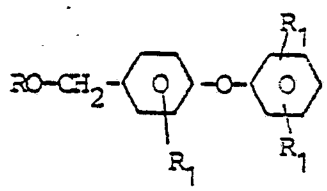

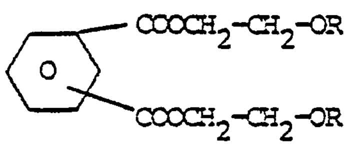

in der ortho-, meta- oder para-Form

Verbindung der allgemeinen Formel:

$R-(O-D-O-X)_n-OD-OR$, wobei HO-D-OH ein Polyol und HO-X-OH eine Dicarbonsäure ist, und zwar jeweils gesättigt oder ungesättigt, cyclisch oder acyclisch.

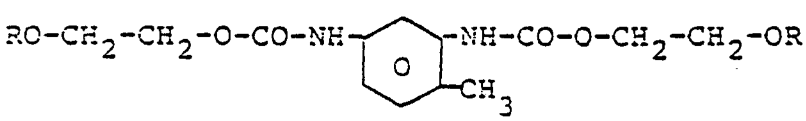

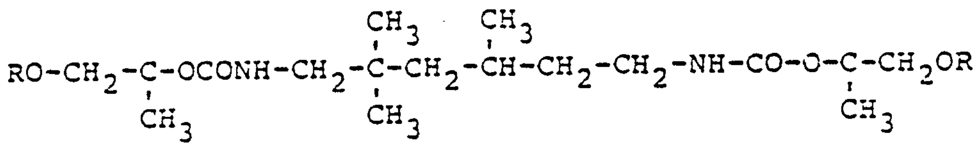

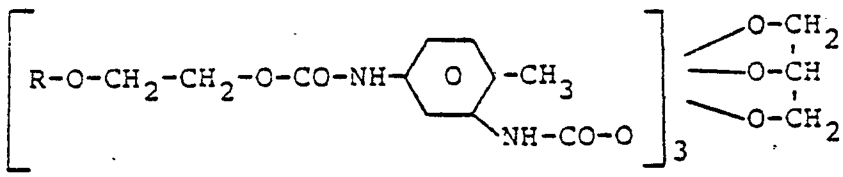

(Durchschnitts-Molgewicht 2500)

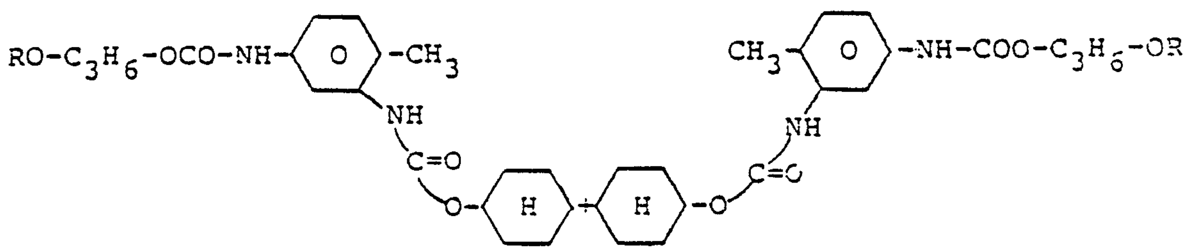

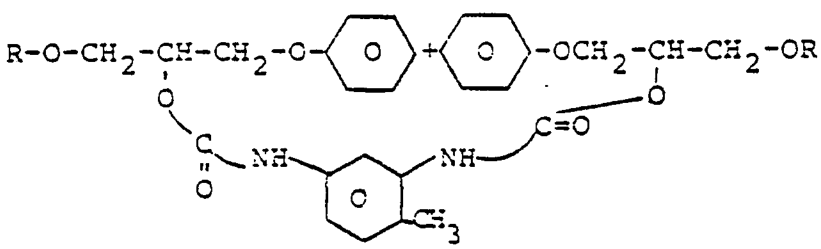

Ferner eignen sich NCO- und Urethangruppen enthaltende Prepolymere, umgesetzt mit Hydroxyalkyl-methacrylat gemäß DT-OS 2 419 887 oder FR-PS 2 271 807.

Die Zahl der in Id eingeführten Oxyalkylgruppen soll 1-10 betragen. Der Vorzugsbereich liegt für Composite-Zahnfüllmassen bei n + m = 1-4, für Überzugs- und Versiegelungsmassen, sowie für Prothesenmaterialien bei n + m = 2-10.

Als Katalysatoren für die Polymerisation der genannten Monomeren oder Monomerengemisch zu den erfindungsgemäßen Dentalmassen sind unter anderem Peroxide oder Azoverbindungen geeignet, insbesondere Benzoylperoxid, o-Toluylperoxid, Chlorbenzoylperoxid, Lauroylperoxid, Cumolhydroperoxid, t-Butylhydroperoxid oder Azodiisobuttersäuredinitril. Soll die Polymerisation bei Raumtemperatur oder im Mund durchgeführt werden, so verwendet man die bekannten Redox-Systeme oder UV-bzw. sichtbares Licht in Kombination mit Photoinitiatoren.

Die Redox-Systeme bestehen bekanntlich aus einem Oxydations- und einem Reaktionsmittel, bei deren Umsetzung freie Radikale gebildet werden. Im allgemeinen verwendet man Peroxide in Kombination mit Aminen, Mercaptanen, Sulfinsäuren, Aminosulfonen oder methylenaktiven Verbindungen, wie N,N-Dimethyl-5-isobutylbarbitursäure. Geeignete Amine sind beispielsweise N,N-Dimethyl-p-toluidin, N,N-Dimethyl-3,5-dimethylanilin, N,N-Bis-hydroxyalkyl-p-toluidin, N,N-Bis-hydroxyalkyl-3,5-dimethylanilin, N,N-Bis-hydroxyalkyl-3,5-di-t-butylanilin oder N,N-Bis-hydroxyalkyl-3,4-dimethylanilin.

Geeignete Photoinitiatoren für die UV-Polymerisation sind beispielsweise Benzoin, Benzoinether, $\alpha$-Acyloximester, Acetophenonderivate wie 2,2-Diethoxyacetophenon, Benzil-dimethylketal, Ester der Phenylglyoxylsäure, halogenierte Aromaten, wie 4-t-Butyl-2,2,2-trichlor-

acetophenon oder 2-Chlorthioanthon, ferner Keton-Amin-Kombinationen, wie Michlers Keton oder Benzophenon + N-Methyldiethanolamin. Solche Keton-Amin-Kombinationen sind beispielsweise in der US-PS 3 759 807 beschrieben.

Die erfindungsgemäßen Mononeren Id sollen vorzugsweise auf dem Dentalgebiet eingesetzt werden, z.B. zur Herstellung von Zahnfüllmitteln, Zahnreparaturmaterialien, Überzugsmassen, Versiegelungsmassen für Kavitäten, Kronen-, Brücken- und Verblendmaterialien, Prothesenmaterialien, künstlichen Zähnen und orthodontischen Vorrichtungen.

Die Darbietungsform hängt vom beabsichtigten Verwendungszweck ab. So können beispielsweise bei der Herstellung von Zahnfüllmaterialien Zwei- oder Einkomponentensysteme verwendet werden. Bei den Zweikomponentensystemen werden die Bestandteile des Redox-Systems auf die beiden Komponenten verteilt, welche in flüssiger oder pastenförmiger Form vorliegen können. Eine pastenförmige Konsistenz wird beispielsweise durch Zumischen von anorganischen oder organischen Füllstoffen, Perlpolymerisaten, oder Pigmenten erzielt. Weitere häufig verwendete Zusatzstoffe sind Antioxydantien zur Verhinderung einer vorzeitigen Polymerisation, UV-Schutzmittel oder Farbstoffe.

Die beiden Komponenten können auch in einer Mischkapsel /siehe beispielsweise DT-PS 1 566 294 oder DT-PS 2 324 296/ getrennt gelagert werden. Vor der Anwendung werden die Komponenten in der Kapsel vereinigt und auf einer Schüttelmaschine vermischt.

Vorteilhafte Einkomponentensysteme enthalten die beanspruchten Monomeren Id, gegebenenfalls Füllstoffe, Farbstoffe und Antioxydantien, ferner Photoinitiatoren für UV- oder sichtbares Licht.

Die Erfindung wird durch die nachfolgenden Beispiele erläutert.

Beispiele

Vorschrift zur Herstellung der oxyalkylierten Bis-hydroxy-methyl-tricyclo-[5.2.0.1.$^{2,6}$]decan Ic (TCD-DM)

Produkt A: TCD-DM + 2 Mol Ethylenoxid

In einem heizbaren Rührautoklaven, der mit einer Vorrichtung zur Azeotrop-Entwässerung versehen ist, werden 4830 g Bishydroxymethyl-tricyclo[5.2.0.1.$^{2,6}$]decan (TCD-DM; Gemische der 3,8-, 3,9- und 4,8-Isomeren) und 600 g Toluol vorgelegt und die Luft gegen Stickstoff ausgetauscht. Man gibt bei 80°C 70 g 50 %ige wäßrige Kaliumhydroxidlösung zu und entfernt bei 100 - 115°C 46 g Wasser aus dem Reaktionsgemisch durch azeotrope Destillation. Anschließend werden bei 100 - 115°C und 0,4 - 0,6 bar 2170 g Ethylenoxid langsam zudosiert und das Gemisch 3 Std. bei 100 - 105°C nachgerührt. Das alkalische Reaktionsprodukt wird mit 700 g Wasser und 245 g einer 12,5 %igen wäßrigen Schwefelsäure neutralisiert. Anschließend wird nach Zugabe eines Filterhilfsmittels und eines Antioxydans, wie 0,05 % 2,6-Bis-t-butyl-p-Kresol das Wasser im Vakuum bei 70-90°C abdestilliert und die abgeschiedenen Salze zusammen mit dem Filterhilfsmittel abfiltriert. Das so erhaltene neutrale Produkt hat eine Hydroxyzahl von 383 und eine Viskosität von $\eta_{25°C}$ = 3550 mPas.

Analog werden folgende Hydroxyalkylierungsprodukte des Bishydroxymethyl-tricyclo-[5.2.0.1.$^{2,6}$]decans (TCD-DM) hergestellt.

Produkt B: TCD-DM + 4 Mol Ethylenoxid,
Einsatz 3688 g TCD-DM + 3312 g Ethylenoxid; OH-Zahl 289;
$\eta_{25°C}$ = 980 mPas.

Produkt C: TCD-DM + 8 Mol Ethylenoxid;
Einsatz 2504 g TCD-DM + 4496 g Ethylenoxid; OH-Zahl 187;
$\eta_{25°C}$ = 480 mPas.

Produkt D: TCD-DM + 2 Mol Propylenoxid;
Einsatz 4398 g TCD-DM + 2602 g Propylenoxid; OH-Zahl 341;
$\eta_{25°C}$ = 5080 mPas.

Produkt E: TCD-DM + 4 Mol Propylenoxid;
Einsatz 3206 g TCD-DM + 3794 g Propylenoxid; OH-Zahl 255;
Viskosität $\eta_{25°C}$ = 1330 mPas.

Beispiel 1

Methacrylsäureester von Produkt A:

1460 g Produkt A, 15 g Methylenblau, 50 g p-Toluolsulfonsäure, 1150 g destillierte Methacrylsäure und 2000 ml Toluol werden unter Einblasen von Luft zum Sieden erhitzt. Das sich bildende Wasser wird durch azeotrope Destillation ausgekreist. Nach 8 Stunden ist die Wasserabspaltung beendet. Man rührt nach dem Abkühlen 1 Std. mit 40 g Bleicherde, um das Methylenblau zu adsorbieren und saugt die blaugefärbte Bleicherde ab. Das Filtrat wird mit überschüssiger Natriumcarbonatlösung bis zum pH-Wert von 8 neutralisiert und über ein Filterhilfsmittel wie Zellstoffmehl filtriert. Die obere Phase wird abgetrennt, mit Natriumchloridlösung gewaschen und nach Zusatz eines Polymerisationsinhibitors im Vakuum eingeengt, bis kein Toluol mehr vorhanden ist.

Ausbeute 1817 g; Verseifungszahl 268 (Th. 262); OH-Zahl 5,6; Viskosität $\eta_{25°C}$ = 104 mPas; Brechzahl $n_D^{20}$ = 1,4925.

Beispiel 2

Methacrylsäureester von Produkt B:

1940 g Produkt B, 15 g Methylenblau, 50 g p-Toluolsulfosäure, 1150 g Methacrylsäure und 2000 ml Toluol werden analog zu Beispiel 1 verestert und aufgearbeitet.

Ausbeute 2364 g; Verseifungszahl 224 (th. 216); OH-Zahl 2,3, Viskosität $\eta_{25°}$ = 118 mPas bei 13 dyn/cm$^2$; Brechzahl $n_D^{20}$ = 1,4880.

Beispiel 3

Methacrylsäureester von Produkt C:

Analog zu Beispiel 1 werden aus 3000 g Produkt C erhalten: 2950 g Methacrylsäureester mit folgenden Kennzahlen: Verseifungszahl 154 (th. 153); OH-Zahl 5,1; Viskosität $\eta_{25°C}$ = 157 mPas; $n_D^{21}$ = 1,4825

Beispiel 4

Methacrylsäureester von Produkt D:

Aus 3280 g Produkt D werden 4051 g des Methacrylesters erhalten; Verseifungszahl 254; Viskosität $\eta_{25°C}$ = 116 mPas; $n_D^{20}$ = 1,4840

Beispiel 5

Methacrylsäureester von Produkt E:

Ais 2600 g Produkt E erhält man 2918 g des entsprechenden Methacrylsäureesters; Verseifungszahl 197 (th. 196), OH-Zahl 9,4; Viskosität $\eta_{25°C}$ = 143 mPas; Brechzahl $n_D^{21}$ = 1,4800

Analog den Beispielen 1-5 können auch die entsprechenden Acrylsäureester hergestellt werden.

Beispiel 6

Es werden mit den nach Beispiel 1 hergestellten Monomeren eine ein aromatisches Amin enthaltende Paste (A-Paste) und eine benzoylperoxidhaltige Paste (B-Paste) hergestellt. Diese beiden Pasten werden zu gleichen Teilen ca. 30 Sekunden innig vermischt, und aus der Mischung werden Probekörper hergestellt, welche bis 37°C gelagert werden. Die physikalischen Eigenschaften der Probekörper wurden gemessen.

Paste 6 A

| | | |
|---|---|---|
| 20,66 | Teile | Monomer nach Beispiel 1 |
| 0,30 | " | N,N-Bis-hydroxypropyl-3,5-dimethylanilin |
| 0,04 | " | Methacryloxypropyl-trimethoxysilan |
| 77,60 | " | feinteilige Lanthan enthaltende Glaskeramik nach DT-PS 2 347 591* vorbehandelt mit 0,4 % Methacryloxypropyl-trimethoxysilan |
| 1,40 | " | Hochdisperse Kieselsäure |

* Die Zusammensetzung der Glaskeramik nach DT-PS 2 347 591 betrug:
4 % $Li_2O$; 3 % ZnO: 15 % $La_2O_3$: 19 % $Al_2O_3$; 46 % $SiO_2$; 5 % $ZrO_2$; 5 % $P_2O_5$; 3 % $Ta_2O_5$;

Paste 6 B

17,7 Teile Monomer nach Beispiel 1
0,36 Teile Benzoylperoxid

| | | |
|---|---|---|
| 0,04 | Teile | Methacryloxypropyl-trimethoxysilan |
| 80,69 | " | gemahlener Bergkristall, vorbehandelt mit 0,04 % Methacryloxypropyl-trimethoxysilan |
| 1,21 | " | Hochdisperse Kieselsäure |

Physikalische Werte der Probekörper

Druckfestigkeit 224 ± 8,5 N/mm²

Biegefestigkeit 103 ± 7,5 MPa

E-Moldul 12975 ± 1447 MPa

Beispiel 7

Analog zu Beispiel 6 wurden mit den Monomeren aus Beispiel 2 folgende Pasten angemischt und daraus Probekörper hergestellt.

Paste 7 A

| | | |
|---|---|---|
| 21,58 | Teile | Monomer aus Beispiel 2 |
| 0,26 | " | bis-propoxyliertes 3,5-Dimethylanilin |
| 0,04 | " | Methacryloxypropyl-trimethoxysilan |
| 76,67 | " | feinteilige Lanthan enthaltene Glaskeramik nach DT-PS 2 347 591, mit 0,4 % Methacryloxypropyl-trimethoxysilan vorbehandelt |
| 1,45 | " | Hochdisperse Kieselsäure |

Paste 7 B

| | | |
|---|---|---|
| 19,59 | Teile | Monomer nach Beispiel 2 |
| 0,40 | " | Benzoylperoxid |

| | | |
|---|---|---|
| 0,20 | Teile | Methacryloxypropyl-trimethoxysilan |
| 78,46 | " | feinteiliger Bergkristall, vorbehandelt mit 0,4 % Methacryloxypropyl-trimethoxysilan |
| 1,35 | " | Hochdisperse Kieselsäure |

Die Druckfestigkeit des Probekörpers betrug 184,2 ± 5,92 $N/mm^2$.

Beispiel 8

Analog zu Beispiel 6 wurden mit dem Monomeren aus Beispiel 3 folgende Pasten angemischt und daraus Probekörper hergestellt.

Paste 8 A

| | | |
|---|---|---|
| 21,53 | Teile | Monomer nach Beispiel 3 |
| 0,30 | " | bis-propoxyliertes 3,5-Dimethylanilin |
| 0,04 | " | Methacryloxypropyl-trimethyloxysilan |
| 76,67 | " | Lanthanhaltige Glaskeramik nach DT-PS 2 347 591, silanisiert |
| 1,46 | " | Hochdisperse Kieselsäure |

Paste 8 B

| | | |
|---|---|---|
| 17,00 | Teile | Monomer nach Beispiel 3 |
| 0,34 | " | Benzoylperoxid |
| 0,04 | " | Methacryloxypropyl-trimethoxysilan |
| 81,13 | " | feinteiliger Bergkristall, vorbehandelt mit 0,4 % Methacryloxypropyl-trimethoxysilan |
| 1,49 | " | Hochdisperse Kieselsäure |

Die aus gleichen Teilen der Paste A und B hergestellten Probekörper hatten folgende physikalische Eigenschaften.
Druckfestigkeit 120,4 ± 6 $N/mm^2$
Biegefestigkeit 21,5 ± 9 MPa

<u>Beispiel 9</u>

Analog zu Beispiel 6 wurde das nach Beispiel 4 hergestellte Monomer zu folgenden Pasten angemischt und daraus Probekörper hergestellt.

Paste 9 A

| | | |
|---|---|---|
| 22,30 | Teile | Monomer nach Beispiel 4 |
| 0,32 | " | bis-propoxyliertes 3,5-Dimethylanilin |
| 0,04 | " | Methacryloxypropyl-trimethoxysilan |
| 0,07 | " | 2-Hydroxy-4-methoxy-benzophenon |
| 76,00 | " | silanisierte La-Glaskeramik nach DT-PS 2 347 591 |
| 1,27 | " | Hochdisperse Kieselsäure |

Paste 9 B

| | | |
|---|---|---|
| 22,32 | Teile | Monomer nach Beispiel 4 |
| 0,46 | " | Benzoylperoxid |
| 0,04 | " | Methacryloxypropyl-trimethoxysilan |
| 0,06 | " | 2-Hydroxy-4-methoxy-benzophenon |
| 75,93 | " | silanisierter feinteiliger Bergkristall |
| 1,19 | " | Hochdisperse Kieselsäure |

| | |
|---|---|
| Druckfestigkeit | 185,5 $N/mm^2$ |
| Biegefestigkeit | 86,8 MPa |
| Durchbiegung | 0,28 mm |
| E-Modul | 10930 MPa |

Beispiel 10

Analog zu Beispiel 6 wurden Probekörper aus folgenden Pasten hergestellt und geprüft. Die Pasten wurden mit dem Monomer nach Beispiel 5 angemischt.

Paste 10 A

| | | |
|---|---|---|
| 20,66 | Teile | Monomer nach Beispiel 5 |
| 0,30 | " | bis-hydroxypropyliertes 3,5-Dimethylanilin |
| 0,04 | " | Methacryloxypropyl-trimethoxysilan |
| 77,60 | " | silanisierte La-Glaskeramik nach DT-PS 2 347 591 |
| 1,40 | " | Hochdisperse Kieselsäure |

Paste 10 B

| | | |
|---|---|---|
| 18,34 | " | Monomer nach Beispiel 5 |
| 0,37 | " | Benzoylperoxid |
| 0,04 | " | Methacryloxypropyl-trimethoxysilan |
| 80,00 | " | feinteiliger silanisierter Bergkristall |
| 1,25 | " | Hochdisperse Kieselsäure |

Durch Vermischen gleicher Gewichtsteile der Pasten 10 A und 10 B wurden Probekörper hergestellt, welche nach 24

Std. Wasserlagerung bei 37°C folgende Prüfwerte zeigten.

| | | |
|---|---|---|
| Druckfestigkeit | 180 ± 11 | N/mm |
| Biegefestigkeit | 94 ± 7 | MPa |
| E-Modul | 10400 ± 785 | MPa |

Beispiel 11

Es wurde ein Monomerengemisch hergestellt durch Vermischen von 80 Teilen des Monomers nach Beispiel 2 mit 20 Teilen Trimethylolpropantrimethacrylat. Die daraus hergestellten Pasten 11 A und 11 B wurden im Verhältnis 1:1 zu Probekörpern verarbeitet.

Paste 11 A

| | | |
|---|---|---|
| 17,20 | Teile | Monomer nach Beispiel 2 |
| 4,30 | " | Trimethylolpropantrimethacrylat |
| 0,30 | " | N,N-Bis-hydroxypropyl-3,5-dimethylanilin |
| 0,04 | " | Methacryloxypropyl-trimethoxysilan |
| 0,06 | " | 2-Hydroxy-4-methoxy-benzophenon |
| 76,64 | " | La-haltige silanisierte Glaskeramik nach DT-OS 2 347 591) |
| 1,46 | " | Hochdisperse Kieselsäure |

Paste 11 B

| | | |
|---|---|---|
| 14,88 | Teile | Monomer nach Beispiel 2 |
| 3,70 | " | Trimethylolpropan-trimethacrylat |
| 0,38 | " | Benzoylperoxid |
| 0,04 | " | Methacryloxypropyl-trimethoxysilan |

| | | |
|---|---|---|
| 0,06 | Teile | 2-Hydroxy-4-methoxy-benzophenon |
| 79,49 | " | silanisierter feinteiliger Bergkristall |
| 1,45 | " | Hochdisperse Kieselsäure |

Druckfestigkeit 198 ± 13 $N/mm^2$
Biegefestigkeit 104 ± 6 MPa
E-Modul 12900 ± 2078 MPa

Beispiel 12

Es wurde ein Monomerengemisch hergestellt aus gleichen Gewichtsteilen des nach Beispiel 3 hergestellten Monomeren und eines Urethan-methacrylats, welches aus 1 Mol Trimethyl-hexamethylen-diisocyanat und 2 Mol ß-Hydroxymethyl-methacrylat hergestellt wurde und nachstehend als Plex 666-1 bezeichent wird.

Paste 12 A

| | | |
|---|---|---|
| 10,79 | Teile | Monomer nach Beispiel 3 |
| 10,79 | " | Plex 666-1 |
| 0,21 | " | N,N-Bis-hydroxypropyl-3,5-dimethylanilin |
| 0,04 | " | Methacryloxypropyl-trimethoxysilan |
| 0,06 | " | 2-Hydroxy-4-methoxy-benzophenon |
| 76,71 | " | silanisierte La-Glaskeramik nach DT-PS 2 347 591 |
| 1,40 | " | Hochdisperse Kieselsäure |

Paste 12 B

| | | |
|---|---|---|
| 10,67 | Teile | Monomer nach Beispiel 3 |
| 10,67 | " | Plex 666-1 |
| 0,45 | " | Benzoylperoxid |
| 0,06 | " | 2-Hydroxy-4-methoxy-benzophenon |
| 0,04 | " | Methacryloxypropyl-trimethoxysilan |
| 76,71 | " | silanisierter feinteiliger Bergkristall |
| 1,40 | " | Hochdisperse Kieselsäure |

Die aus gleichen Teilen der Pasten 12 A und 12 B hergestellten Probekörper hatten nach 24-stündiger Wasserlagerung folgende Festigkeiten.

Druckfestigkeit 198 ± 14 $N/mm^2$
Biegefestigkeit 94 ± 4 MPa
E-Modul 6956 ± 135 MPa

## Beispiel 13

Zum Einsatz gelangt ein Monomerengemisch aus 72 Teilen Monomer nach Beispiel 5 und 28 Teilen Trimethylolpropantrimethacrylat. Es werden analog zu Beispiel 6 die Pasten 13 A und 13 b angemischt und im Gewichtsverhältnis 1:1 zu Probekörpern ausgehärtet.

Paste 13 A

| | | |
|---|---|---|
| 16,05 | Teile | Monomer nach Beispiel 5 |
| 6,23 | " | Trimethylolpropantrimethacrylat |

| | | |
|---|---|---|
| 0,33 | Teile | N,N-Bis-Hydroxypropyl-3,5-dimethylanilin |
| 0,04 | " | Methacryloxypropyl-trimethoxysilan |
| 75,76 | " | silanisierte La-haltige Glaskeramik nach DT-PS 2 347 591 |
| 1,52 | " | Hochdisperse Kieselsäure |

Paste 13 B

| | | |
|---|---|---|
| 13,38 | Teile | Monomer nach Beispiel 5 |
| 5,20 | Teile | Trimethylolpropan-trimethacrylat |
| 0,38 | " | Benzoylperoxid |
| 0,04 | " | Methacryloxypropyl-trimethoxysilan |
| 0,06 | " | 2-Hydroxy-4-methoxy-benzophenon |
| 79,49 | " | silanisierter feinteiliger Bergkristall |
| 1,45 | " | Hochdisperse Kieselsäure |

Druckfestigkeit 172 ± 17 $N/mm^2$
Biegefestigkeit 86 ± 23 MPa
E-Moldul 10866 ± 702 MPa

Beispiel 14

Beispiel 6 wurde wiederholt mit einer Mischung aus 70 Teilen Monomer 5 und 30 Teilen 2,2-Propan-bis/3-(4-phenoxy)-1,2-dihydroxypropan-1-methacrylat7. Das letztgenannte Monomer wird auch als BIS-GMA bezeichnet.

Paste 14 A

| | | |
|---|---|---|
| 17,70 | Teile | Monomer aus Beispiel 5 |
| 4,70 | " | BIS-GMA |

| | | |
|---|---|---|
| 0,23 | Teile | N,N-Bis-hydroxypropyl-3,5-dimethylanilin |
| 0,04 | " | Methacryloxypropyl-trimethoxysilan |
| 0,06 | " | 2-Hydroxy-4-methoxy-benzophenon |
| 76,00 | " | La-haltige Glaskeramik nach DT-PS 2 347 591, vorbehandelt mit 0,4 % Methacryloxypropyl-trimethylsilan |
| 1,27 | " | Hochdisperse Kieselsäure |

Paste 14 B

| | | |
|---|---|---|
| 15,10 | Teile | Monomer nach Beispiel 5 |
| 6,50 | " | BIS-GMA |
| 0,43 | " | Benzoylperoxid |
| 0,04 | " | Methacryloxypropyl-trimethoxysilan |
| 0,06 | " | 2-Hydroxy-4-methoxy-benzophenon |
| 76,72 | " | feinteiliger Bergkristall, vorbehandelt mit 0,4 % Methacryloxypropyl-trimethoxysilan |
| 1,15 | " | Hochdisperse Kieselsäure |

Die aus gleichen Teilen der Pasten 14 A und 14 B hergestellten Probekörper hatten folgende physikalische Eigenschaften.

Druckfestigkeit 180,9 $N/mm^2$
Biegefestigkeit 77,9 MPa
Durchbiegung 10680 MPa

__Beispiel 15__

Beispiel 6 wurde wiederholt mit einer Mischung aus je 50 Teilen des Monomers nach Beispiel 1 und 50 Teilen

eines Urethanmethacrylates, welches aus 1 Mol Trimethylhexamethylendiisocyanat und 2 Mol ß-Hydroxy-ethyl-methacrylat hergestellt worden war. (Plex 666-1). Das Monomerengemisch wurde zusätzlich mit 0,2 % Methacryloxypropyltrimethoxysilan und 0,3 % 2-Hydroxy-4-methoxybenzophenon versetzt.

Paste 15 A

| | | |
|---|---|---|
| 22,50 | Teile | Monomerengemisch |
| 0,23 | " | N,N-Bis-hydroxypropyl-3,5-dimethylanilin |
| 76,00 | " | silanisierte La-haltige Glaskeramik nach DT-PS 2 347 591 |
| 1,27 | " | Hochdisperse Kieselsäure |

Paste 15 B

| | | |
|---|---|---|
| 22,40 | Teile | Monomerengemisch |
| 0,46 | " | Benzoylperoxid |
| 0,02 | " | 2,6-Di-tert.-butyl-4-kresol |
| 75,93 | " | silanisierter feinteiliger Bergkristall |
| 1,19 | " | Hochdisperse Kieselsäure |

Die aus gleichen Teilen der Pasten 15 A und 15 B hergestellten Probekörpern hatten folgendes Festigkeitsniveau:

| | |
|---|---|
| Druckfestigkeit | 196,7 $N/mm^2$ |
| Biegefestigkeit | 87,6 MPa |
| E-Modul | 11700 MPa |

Beispiel 16

Beispiel 6 wurde wiederholt mit einer Mischung aus 70 Teilen des nach Beispiel 1 hergestellten Monomeren und 30 Teilen BIS-GMA. Das Gemisch wurde zusätzlich mit 0,2 % Methacryloxypropyl-trimethoxysilan, 0,3 % 2-Hydroxy-4-methoxy-benzophenon und 0,075 % 2,6-di-tert.-butyl-4-kresol versetzt.

Paste 16 A

| | | |
|---|---|---|
| 22,50 | Teile | stabilisiertes Monomerengemisch |
| 0,23 | " | N,N-Bis-hydroxypropyl-3,5-dimethylanilin |
| 76,00 | " | La-haltige silanisierte Glaskeramik nach DT-PS 2 347 591 |
| 1,27 | " | Hochdisperse Kieselsäure |

Paste 16 B

| | | |
|---|---|---|
| 22,42 | Teile | stabilisiertes Monomerengemisch |
| 0,46 | " | Benzoylperoxid |
| 75,93 | " | silanisierter feinteiliger Bergkristall |
| 1,19 | " | Hochdisperse Kieselsäure |

die Festigkeitswerte der aus Paste 16 A und 16 B hergestellten Probekörper

Druckfestigkeit 192,5 $N/mm^2$

Biegefestigkeit 80,0 M/Pa

E-Modul 12420 MPa

Beispiel 17

Beispiel 6 wurde wiederholt mit einem Gemisch aus gleichen Teilen Monomer nach Beispiel 2 und dem Reaktionsprodukt aus 1 Mol Trimethyl-hexamethylen-diisocyanat und 2 Mol ß-Hydroxy-ethyl-methacrylat. Das Gemisch wurde vor der Pastenherstellung noch mit Methacryloxypropyl-trimethoxysilan und 0,3 % 2-Hydroxy-4-methoxybenzophenon versetzt.

Paste 17 A

| | | |
|---|---|---|
| 22,50 | Teile | stabilisiertes Monomerengemisch |
| 0,23 | " | Bis-Hydroxypropyl-3,5-dimethylanilin |
| 76,00 | " | silanisierte La-haltige Glaskeramik |
| 1,27 | " | Hochdisperse Kieselsäure |

Paste 17 B

| | | |
|---|---|---|
| 22,42 | Teile | Monomerengemisch |
| 0,01 | " | 2,6-Di-tert.-butyl-4-Kresol |
| 0,45 | " | Benzoylperoxid |
| 75,93 | " | silanisierter feinteiliger Bergkristall |
| 1,19 | " | Hochdisperse Kieselsäure |

Gleiche Gewichtsteile der Pasten 17 A und 17 B wurden zu Probekörpern verarbeitet.

Druckfestigkeit 178,5 $N/mm^2$
Biegefestigkeit 91,3 MPa
E-Modul 10710 MPa

Beispiel 18

Beispiel 6 wurde wiederholt mit einem Gemisch aus 70 Teilen Monomer nach Beispiel 2 und 30 Teilen BIS-GMA; das Gemisch enthielt zusätzlich 0,2 % Methacryloxypropyl-trimethoxysilan, 0,3 % 2-Hydroxy-4-methoxy-benzophenon und 0,05 % 2,6-Di-tert.-butyl-4-kresol.

Paste 18 A

| | | |
|---|---|---|
| 22,50 | Teile | stabilisiertes Monomerengemisch |
| 0,23 | " | N,N-Bis-hydroxypropyl-3,5-dimethylanilin |
| 76,00 | " | silanisierte La-haltige Glaskeramik nach DT-PS 2 347 591 |
| 1,27 | " | Hochdisperse Kieselsäure |

Paste 18 B

| | | |
|---|---|---|
| 22,42 | Teile | stabilisiertes Monomerengemisch |
| 0,46 | " | Benzoylperoxid |
| 75,93 | " | silanisierter feinteiliger Quarz |
| 1,19 | " | Hochdisperse Kieselsäure |

Die aus gleichen Gewichtsmengen der Pasten 18 A und 18 B hergestellten Probekörper hatten folgende Festigkeitswerte:

Druckfestigkeit 188,6 $N/mm^2$

Biegefestigkeit 76,2 MPa

E-Modul 10720 MPa

Beispiel 19

Zur Herstellung einer UV-härtbaren röntgenopaken Zahnfüllmasse wurde aus folgenden Komponenten eine Paste angemischt:

| | | |
|---|---|---|
| 22,73 | Teile | Monomer nach Beispiel 4, enthaltend 0,2 % Methacryloxypropyl-trimethoxysilan |
| 0,28 | " | Benzil-dimethylketal |
| 37,78 | " | silanisierte La-haltige Glaskeramik nach DT-PS 2 347 591 |
| 37,78 | " | silanisierte Glaskeramik auf Basis my-Cordierit |
| 1,43 | " | Hochdisperse Kieselsäure |

Bei Bestrahlung mit einem handelsüblichen UV-Polymerisationsgerät wird nach 20 Sekunden eine Polymerisationstiefe von 2,2 mm erreicht.
Die Druckfestigkeit eines Probekörpers betrug 203,0 $N/mm^2$.

Beispiel 20 (Vergleichsversuch)

Zum Vergleich wurden Pasten hergestellt mit dem nach der DT-PS 2 816 823, Beispiel 1, hergestellten Methacrylsäureester des Bishydroxymethyl-tricyclo-[5.2.1.$0^{2.6}$]-decans. Das Monomere wurde mit 0,2 % Methacryloxypropyltrimethoxysilan und 0,3 % 2-Hydroxy-4-methoxy-benzophenon stabilisiert und zu folgenden Pasten verarbeitet:

Paste 20 A

22,40 Teile TCD-DM-Methacrylat stabilisiert

| | | |
|---|---|---|
| 0,32 | Teile | N,N-Bis-hydroxypropyl-3,5-dimethylanilin |
| 75,76 | " | La-haltige Glaskeramik nach DT-PS 2 347 591, vorbehandelt mit 0,4 % Methacryloxypropyl-trimethoxy-silan |
| 1,52 | " | Hochdisperse Kieselsäure |

Paste 20 B

| | | |
|---|---|---|
| 18,67 | Teile | TCD-DM-Methacrylat stabilisiert |
| 0,39 | " | Benzoylperoxid |
| 79,49 | " | mit 0,4 % Methacryloxypropyl-trimethylsilan vorbehandelter feinteiliger Bergkristall |
| 1,45 | " | Hochdisperse Kieselsäure |

Durch Vermischen gleicher Teile der beiden Pasten 20 A und 20 B werden Probekörper hergestellt, die in ca. 3 Minuten aushärten. Nach 24-stündiger Lagerung bei 37°C in Wasser werden folgende physikalische Werte gefunden:

Druckfestigkeit 216,7 ± 28,3 N/mm²
Biegefestigkeit 82,8 ± 17,9 MPA
Durchbiegung 0,19 ± 0,02
E-Modul 15300 ± 2107 MPa

Patentansprüche:

1. Dentalmassen, enthaltend polymerisierbare (Meth)-acrylsäureester der allgemeinen Formel I

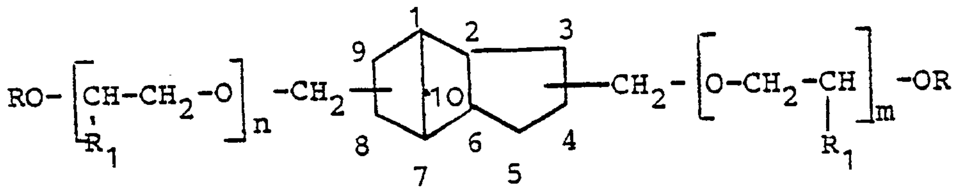

(I)

in welcher

R für $CH_2=CH-CO-$ oder $CH_2=\underset{CH_3}{C}-CO-$,

$R_1$ für H, $CH_3$ oder $C_2H_5$ und

n + m für 1 - 10

stehen, sowie gegebenenfalls Füllstoffe, Stabilisatoren und Pigmente.

2. Dentalmassen, enthaltend polymerisierbare (Meth)-acrylsäureester der allgemeinen Formel (II)

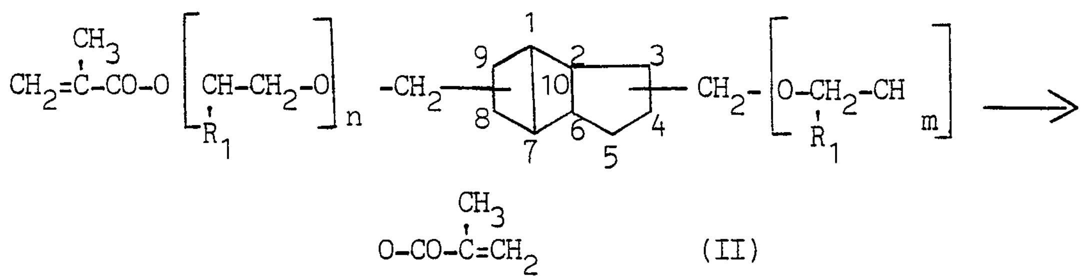

(II)

in welcher

$R_1$ für H, $CH_3$ oder $C_2H_5$ und

n + m für 1 - 10

stehen, sowie gegebenenfalls Füllstoffe, Stabilisatoren und Pigmente.

3. Dentalmassen, enthaltend polymerisierbare (Meth)-acrylsäureester der allgemeinen Formel (III)

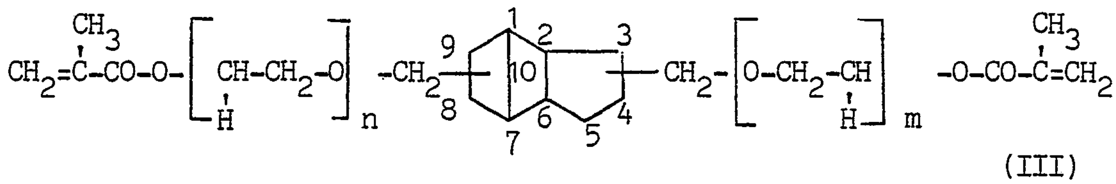

(III)

in welcher n + m für 1 - 10 stehen,
sowie gegebenenfalls Füllstoffe, Stabilisatoren und Pigmente.

4. Dentalmassen, enthaltend polymerisierbare (Meth)-acrylsäureester der allgemeinen Formel (IV)

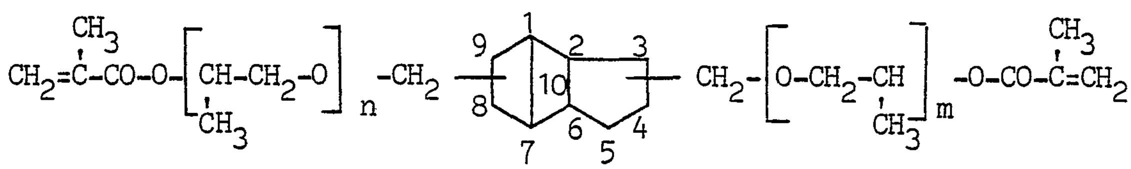

(IV)

in welcher n + m für 1 - 10 stehen,
sowie gegebenenfalls Füllstoffe, Stabilisatoren und Pigmente.

5. Dentalmassen gemäß den Beispielen.

6. Dentalmassen nach Anspruch 1, enthaltend polymerisierbare (Meth)acrylsäureester der allgemeinen Formel (I) in Mischung mit anderen bekannten polymerisierbaren Di(meth)-acrylsäureestern.

7. Dentalmassen nach Anspruch 1, enthaltend polymerisierbare (Meth)acrylsäureester der allgemeinen Formel I in Mischung mit anderen bekannten polyfunktionellen Monomeren.

8. Dentalmassen nach Anspruch 2, enthaltend polymerisierbare (Meth)acrylsäureester der allgemeinen Formel II in Mischung mit anderen bekannten polymerisierbaren Di(meth)-acrylsäureestern.

9. Dentalmassen nach Anspruch 3, enthaltend polymerisierbare (Meth)acrylsäureester der allgemeinen Formel III in Mischung mit anderen bekannten polymerisierbaren Di(meth)-acrylsäureestern.

10. Dentalmassen nach Anspruch 4, enthaltend polymerisierbare (Meth)acrylsäureester der allgemeinen Formel IV in Mischung mit anderen bekannten polymerisierbaren Di(meth)-acrylsäureestern.